(19) Europäisches Patentamt
European Patent Office
Office européen des brevets

(11) Publication number: **0 520 103 A1**

(12) **EUROPEAN PATENT APPLICATION**

(21) Application number: **91305793.1**

(22) Date of filing: **26.06.91**

(51) Int. Cl.5: **C08F 36/04**, C08F 4/30, C08F 2/06

(43) Date of publication of application: **30.12.92 Bulletin 92/53**

(84) Designated Contracting States: **DE FR GB**

(71) Applicant: **TEXACO CHEMICAL COMPANY**
**3040 Post Oak Boulevard**
**Houston, Texas 77056(US)**

(72) Inventor: **Knifton, John Frederick**
**10900 Catskill Trail**
**Austin, Texas 78758(US)**
Inventor: **Marquis, Edward Thomas**
**9004 Collinfield Drive**
**Austin, Texas 78758(US)**

(74) Representative: **Brock, Peter William et al**
**UROUHART-DYKES & LORD 91 Wimpole Street**
**London W1M 8AH(GB)**

(54) **Synthesis of hydroxyl-terminated diene oligomers.**

(57) Hydroxyl-terminated diene oligomers, and especially polybutadiene oligomers with a high OH functionality may be formed, with little equipment fouling arising from solid or gel-type by-products, by polymerizing a diene, preferably 1,3-butadiene in the presence of an aqueous solution of hydrogen peroxide employing a glycol monoalkyl ether or glycol monoalkylether carboxylate as solvent.

Preferred solvents have the formula

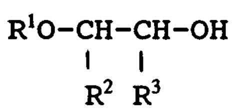

or

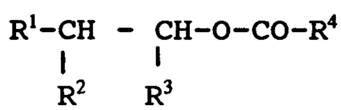

wherein $R^1$ and $R^4$ are $C_1$-$C_{10}$ alkyl, and $R^2$ and $R^3$ are hydrogen or methyl.

EP 0 520 103 A1

This invention relates to the synthesis of hydroxyl-terminated diene oligomers, such as polybutadienes.

More particularly this invention is directed to an improvement in a process for polymerizing dienes, such as 1,3-butadiene, with selective formation of relatively low molecular weight hydroxyl-terminated oligomers, in the presence of an aqueous solution of hydrogen peroxide. In the process of the invention, oligomers having a high degree of OH functionality are obtained, with little or no formation of solid or gel-type insoluble rubber by-products, by using a solvent selected from aliphatic glycol monoalkyl ethers and aliphatic glycol monoalkyl ether carboxylates.

Low-molecular weight homopolymers and copolymers of 1,3,-dienes have been known for a long time. It is advantageous for many uses to alter the properties of the hydrophobic polymers, in a controlled fashion, by the introduction of polar groups. One such group is the hydroxyl group, because it reacts, for example, with isocyanates.

It is also known that hydroxyl-terminated polybutadiene can provide polyurethanes having good hydrolytic stability, chemical resistance and a wide range of mechanical properties.

Processes for the preparation of polyhydroxybutadiene (hydroxyl-containing butadiene homopolymers) are known in the art, and may be prepared, for example, by the methods described in US-A-2877212, -3055952, -3333015, -3338861, -3427366, -3673168 and -3796762. For a review of polymeric butadienes see J. N. Henderson, Encyl. Polym. Sci. Eng. 515-36, 2 (1985) and W. Heitz, Telechelic Polymers: Synthesis and Applications, Chapter 4, p.61 (1989).

US-A-4460801 discloses a process for reacting butadiene and water in the presence of a palladium salt and boric acid in a polar, aprotic solvent to prepare an unsaturated fatty alcohol of the formula:

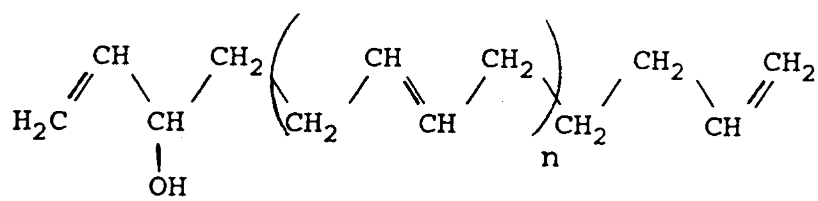

(A)

where n = 2 or 4.

US-A-4670518 discloses a process for the production of low-molecular weight homopolymers and/or copolymers of 1,3-dienes carrying hydroxymethyl groups partially esterified with formic acid, by reacting 1,3-dienes having an average molecular weight of 500 to 8000 with formaldehyde at temperatures of 150 to 300 ° C, optionally in the presence of a solvent and stabilizer.

Butadiene has been polymerized with tetravalent molybdenum catalysts. See M. Zhao et al., C.A. Selects, p.12, 19 (1986). In other work, alpha, omega- hydroxyl-terminated polybutadienes, having different molecular weights and microstructures, were prepared in nonpolar media using lithium-naphthalene-THF as a catalyst; See US-A-3055952 and DE-A-1173658.

US-A-4721754 describes a polybutadiene composition useful for the preparation of polyurea and/or polyurethane elastomers, comprising a blend of a polyhydroxybutadiene homopolymer and an amine-terminated polybutadiene.

A problem has existed in polybutadiene solvent recovery units, wherein solids formation below the feed trays of the distillation columns, may lead to fouling which plugs the trays and downcomers. The fouling is apparently caused by the presence of hydrogen peroxide, low molecular weight polybutadienes, and polymeric precursors, such as butadiene and vinylcyclohexene formed by the polymerization reaction. Such fouling may occur approximately every 5 to 6 days at high plant production rates, thus requiring frequent and expensive shutdowns for cleaning.

US-A-4518770 discloses a method of reducing the fouling in a distillation unit, which comprises injecting into the distillation unit, along with the feed stream from which the polyhydroxybutadiene has been removed, an aqueous solution of an alkali metal sulphite or bisulphite.

JP-A-1012707 (Idemitsu Petrochem) discloses a method for preparing a liquid diene polymer containing OH groups by reacting the conjugated diene monomer with $H_2O_2$ in the presence of a carboxylic acid.

It would be extremely desirable in the art if a process were available to provide, low molecular weight oligomers which possess a high degree of OH functionality while avoiding the formation of solid or gel-type insoluble rubbers, which are known to foul the reactor.

One embodiment of this invention provides a process for the preparation of hydroxyl-terminated diene oligomers, preferably polybutadiene oligomers by polymerizing a diene, preferably 1,3-butadiene, in the presence of an aqueous solution of hydrogen peroxide. According to the invention, a glycol monoalkyl ether

or glycol monoalkyl ether carboxylate is employed as solvent.

According to one embodiment, the solvent is a glycol ether of the formula

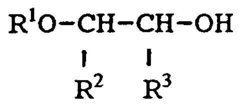

(I)

wherein $R^1$ is $C_1$-$C_{10}$ alkyl, and $R_2$ and $R^3$ are each hydrogen or methyl.

Preferred glycol ethers are propylene glycol t-butyl ether, 2-methoxyethanol, 2-ethoxyethanol, 2-isopropoxyethanol, 2-t-butoxyethanol or diethylene glycol monobutyl ether.

According to another embodiment, the solvent is a glycol monoalkyl ether carboxylate having the formula

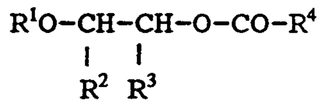

(II)

wherein $R^1$, $R^2$ and $R^3$ have the meanings given above, and $R^4$ is $C_1$-$C_{10}$ alkyl.

In this embodiment, the solvent is preferably an alkylene glycol monoalkyl ether acetate, most preferably ethylene glycol monomethyl ether acetate or propylene glycol monomethyl ether acetate.

This invention demonstrates an improvement over the prior art, in that the hydroxyl-terminated diene oligomers possess low molecular weight and, at the same time, a high OH functionality. Even more important is the improvement demonstrated by the process of the invention that allows for the formation of the desired oligomers without the accompanying formation of solid or gel-type insoluble rubbers that may foul the reaction system.

The process has the following advantages: 1) the solvent can be easily removed from the system; 2) it does not interfere with the polymerization process; 3) it has miscibility with a dilute aqueous solution of hydrogen peroxide over a wide range of weight ratios; 4) it provides a product with desirable properties; and 5) it minimizes polymer build-up in the reactor system in the form of gel or solid rubber-like oligomers.

The products from 1,3-butadiene can be represented by the following structure:

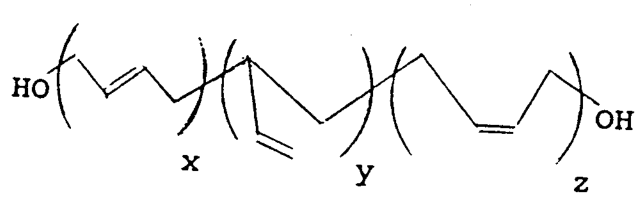

(III)

where x, y and z are numbers.

The hydroxyl-terminated diene oligomers prepared according to this invention contain hydroxyl groups that are in predominantly primary, terminal positions on the main hydrocarbon chain and are allylic in configuration. Ordinarily, at least 1.8 hydroxyl groups are present per molecule on the average, and advantageously there are from 2.1 to 3, preferably 2.1 to 2.8, hydroxyls per polymer molecule. The diene polymer has most of its unsaturation in the main hydrocarbon chain, such that x plus z in the general structure (III) is greater than y. This formula (III) should not be understood as implying that the polymers are necessarily in blocks, but the cis-1,4-, trans-1,4- and vinyl-(1,2) unsaturation are usually distributed throughout the polymer molecule. For example, x may represent a number sufficient to give a trans-1,4-unsaturation content of 40 to 70 percent; y may be a number sufficient to provide a 1,2-vinylic unsaturation content of 10 to 35 percent and z may be sufficient to provide cis-1,4-unsaturation of 10 to 30 percent. Often the polymer will contain largely trans-1,4-units, e.g. 50 to 65 percent, with 15 to 25 percent cis-1,4-units, and 15 to 25 percent 1,2-units. Branching may also occur in the above polymers, especially those prepared at higher temperatures. Ether and carbonyl linkages may appear in the lower molecular weight

oligomer fractions.

The number average molecular weight of the product oligomers, e.g. of general structure (III) is ordinarily from 100 to 20,000, and the hydroxyl content is from 0.1 to 20 meq/g, and may be higher. Preferably, the number average molecular weight is from 200 to 2000 and the hydroxyl content is from 1 to 10 meq/g. Product oligomers of this type are illustrated by the accompanying Examples.

Preparation of the product of this invention may be carried out typically by combining a 10 to 80%, more preferably a 30 to 70%, aqueous solution of hydrogen peroxide with a glycol monoalkyl ether or glycol monoalkyl ether carboxylate solvent, and then reacting the liquid mixture with the diene, preferably at a pressure of 0.1 to 35 MPa and a temperature of 50 to 200° C, to form a one or two layered product, and subsequently stripping said product or products to remove lights, solvents, etc.

The dienes which are employed to prepare the polyhydroxydienes include unsubstituted, 2-substituted or 2,3-disubstituted 1,3-dienes, preferably having 4 to 12 carbon atoms. The diene preferably has 4 to 6 carbon atoms, and the substituents in the 2- and/or 3-position may be hydrogen, alkyl, e.g. alkyl, having 1 to 4 carbon atoms), aryl (substituted or unsubstituted), halogen, nitro, nitrile etc. Typical dienes which may be employed are 1,3-butadiene, isoprene, chloroprene, 2-cyano-1,3-butadiene, 2,3-dimethyl-1,3-butadiene, 2-phenyl-1,3-butadiene, 2-methyl-3-phenyl-1,3-butadiene, etc. The examples demonstrate the particular effectiveness of 1,3-butadiene.

The aqueous solution of hydrogen peroxide is diluted with water. The $H_2O_2$ content can be from 10 to 80%. Preferably the aqueous solution comprises 30 to 50% of $H_2O_2$. Lower contents can be used if necessary.

The molar ratio of hydrogen peroxide feed to diene may be from 1:100 to 100:1 or even higher, but in order to prepare desired, highly functionalized, OH-oligomers of low molecular weight, the initial $H_2O_2$: diene ratio should preferably be in the range of 1:10 to 10:1 for an economically attractive process; most preferred are molar ratios of ca. 1:1.

The solvent is the critical factor in the improvement of this invention. The solvent should be able to solubilize the aqueous hydrogen peroxide, the diene, e.g. butadiene and the hydroxyl-terminated polymer into a single phase over a wide range of reactant/product ratios. In addition the solvent should be able to solubilize adequately the diene at the temperature of oligomerization (e.g. 50 to 200° C) in order to ensure that there is no polymer build up in the diene entrance lines and other cooler portions of the reactor system. It is desirable that the solvent should not interfere with the polymerization process nor be incorporated into the polymer product. Such a solvent system would have great commercial potential, especially if the solvent were of low cost and has a low enough boiling point, so that it can be easily stripped from the desired OH-oligomer product.

It has been surprisingly discovered according to one embodiment of the invention, that certain glycol monoalkyl ethers provide all these advantages in the polymerization process. Preferred ethers have the formula (I) as defined above. The glycol ethers can be aromatic or aliphatic glycol ethers. Aliphatic glycol monoalkyl ethers which work in the process include 2-methoxyethanol, 2-ethoxyethanol, propylene glycol mono-t-butyl ether, diethylene glycol monobutyl ethers, diethylene glycol monomethyl ether, 2-isopropoxyethanol and 2-t-butoxyethanol. Aromatic glycol ethers which may be suitable include 2-phenoxyethanol and p-methoxyphenoxyethanol.

Commercially available glycols which can be used include ARCOSOLV$^R$ PTB. ARCOSOLV$^R$ PTB is the tradename for propylene glycol mono-t-butyl ether, marked by Arco Corp. Also suitable are cellosolve solvents from Union Carbide, such as methyl cellosolve, formula $CH_3OCH_2CH_2OH$ and butyl cellosolve, $C_4H_9OCH_2CH_2OH$, as well as PROPASOL solvents such as PROPASOL solvent P having the formula $C_3H_7OCH_2CH(CH_3)OH$.

Examples 1 and 2 demonstrate that good results have been achieved with propylene glycol mono-t-butyl ether and 2-methoxyethanol.

It has also been surprisingly discovered according to another embodiment of the invention, that certain glycol ether carboxylate solvents, particularly alkylene glycol monoalkyl ether acetates provide the above-mentioned advantages in the polymerization process. The glycol ether acetates can be aromatic or aliphatic glycol ether acetates. Aliphatic glycol monoalkyl ether acetates which work in this process include ethylene glycol monoalkyl ether acetates and carboxylates having the general structure (II) as defined above.

Preferred compounds of formula (II), are compounds of the formula

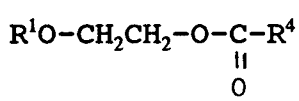

where $R^1$ and $R^4$ are alkyl radicals having 1 to 10, preferably 1 to 6, carbon atoms that may, or may not, be different, but which are typically methyl, ethyl, isopropyl, t-butyl and n-hexyl. Typical examples include ethylene glycol monomethyl ether acetate, ethylene glycol monoethyl ether acetate, ethylene glycol monomethyl ether propionate, ethylene glycol monobutyl ether acetate, ethylene glycol mono-t-butyl ether acetate, ethylene glycol monopropyl ether acetate and ethylene glycol monohexyl ether acetate.

Typical examples of propylene glycol compounds include propylene glycol monomethyl ether acetate, propylene glycol monobutyl ether acetate or propylene glycol mono-t-butyl ether acetate. These propylene glycol monoalkyl ether acetates or carboxylates may, or may not, be a mixture of isomeric forms.

Suitable aromatic glycol ether acetates include 2-phenoxylethyl acetate and p-methoxyphenoxyethyl acetate.

Generally, the aliphatic glycol ether carboxylates suitable for the practice of this invention are made from aliphatic carboxylic acids with up to 6 carbon atoms. Examples include acetic acid, propionic acid and the butyric acids.

Examples 3 and 4 herein demonstrate that good results have been achieved with ethylene glycol monomethyl ether acetate and propylene glycol monomethyl ether acetate.

While the hydroxyl components of the product polyhydroxydienes are predominantly primary, terminal and allylic in structure, the ratio of cis-1,4, trans- 1,4 and 1,2-vinyl unsaturation which occurs in the diene polymers prepared by this invention, in addition to the number and location of the hydroxyl groups, an the molecular weight of the polymers, can be a function of polymerization conditions, particularly the temperature, the $H_2O_2$ to feed ratio, and the type of addition polymerization system employed in forming the polymer. It has been found that diene polymers of the desired configuration can be obtained using hydrogen peroxide as the initiator for polymerization in a mutual solvent system.

This free-radical addition polymerization usually takes place in solution at a temperature of 50 to 200 °C.

The polymerization may be conducted batchwise in a continuous slurry reactor, or in a stirred tank, continuous flow reactor.

Polymerization of dienes, preferably 1,3-butadiene, in the presence of about 50% $H_2O_2$ can generally be conducted at temperatures from 100 to 150 °C. The operating pressure may be from 0.1 to 35 MPa. The most preferred temperature range is 110 to 130 °C and the preferred pressure range is 0.79 to 7 MPa.

Example 1 illustrates the synthesis of desired low molecular weight hydroxyl-terminated polybutadiene oligomers having a high degree of hydroxyl functionality using hydrogen peroxide as initiator and propylene glycol t-butyl ether as solvent.

Advantages to using the solvent include:

a) Miscibility with 50% aqueous hydrogen peroxide over a wide range of weight ratios.

b) The ability to solubilize 1,3-butadiene at the temperature of oligomerization so as to ensure no polymer build-up in the butadiene feed lines or the cooler parts of the reactor system. This polymer may be formed in other solvent systems as a gel or solid rubber-like oligomer.

c) Lack of interference with the polymerization process or the formation of desired hydroxyl-terminated polybutadiene oligomer.

d) The solvent has a relatively low b.p (151 °C) and can easily be stripped from the desired oligomer product.

Example 2 illustrates the synthesis of desired low molecular weight hydroxyl-terminated polybutadiene oligomers of structure A using 2-methoxyethanol (ethylene glycol monomethyl ether) as the solvent of choice.

Example 3 illustrates the synthesis of desired low molecular weight hydroxyl-terminated polybutadiene oligomers having a high degree of hydroxyl functionality using hydrogen peroxide as initiator and ethylene glycol monoethyl ether acetate as solvent.

Advantages to using this solvent include

a) Its miscibility with 50% aqueous hydrogen peroxide over a wide range of weight ratios.

b) It does not interface with the polymerization process or the formation of desired hydroxyl-terminated polybutadiene oligomer.

c) It can be easily removed from the desired oligomer product.

Example 4 illustrates the synthesis of desired low molecular weight hydroxyl-terminated polybutadiene oligomers of the same type using propylene glycol monomethyl ether acetate as the solvent of choice.

## EXAMPLE 1

A 300g capacity stirred, autoclave fitted with temperature and pressure controls plus facilities for two

continuous feed additions was charged with propylene glycol mono-t-butyl ether and then heated to temperature (120°C) under pressure (4.24 MPa). A liquid mixture of 50% hydrogen peroxide aqueous solution (2 parts) and propylene glycol mono-t-butyl ether (3 parts) was then fed continuously into the autoclave at a rate of 300g/h (1.76 moles $H_2O_2$/h), and when the system was lined out, a second feed of 1,3-butadiene was also introduced simultaneously at the rate of 100g/h (1.85 moles/h). After a few hours on stream, typical liquid product was collected under these steady state conditions for about 30 hours.

The two-layer liquid product was allowed to stand and the two layers were separated. A sample (1503g) of the top layer was stripped to remove lights, solvents, etc., and the residual, water white liquid (895g) had the following analysis.

| | |
|---|---|
| Number Average MW | 1303 |
| Weight Average MW | 2283 |
| Dispersity | 1.8 |
| Viscosity | 3284 cs/25°C |
| Hydroxyl Number | 1.9 meq KOH/g |

A sample (8484g) of the heavier layer was likewise stripped to remove lights, solvent, etc., and the residual liquid (1058g) had the analysis.

| | |
|---|---|
| Number Average MW | 312 |
| Weight Average MW | 871 |
| Dispersity | 2.8 |
| Viscosity | 7634 cs/25°C |
| Hydroxyl Number | 8.7 meq KOH/g |

$^{13}C$ NMR analysis of the top layer products shows the total carbon associated with the three major functionalities of this material, (i.e. olefin, OH and aliphatic) to be about 48, 4.4 and 48%, respectively.

EXAMPLE 2

Using the same autoclave reactor system as Example 1, as well as the same start-up procedures, the autoclave was charged with a mixture of 50% hydrogen peroxide solution (2 parts) and 2-methoxyethanol (3 parts) at a rate of 300g/h (1.76 moles $H_2O_2$/h) plus 1,3-butadiene at 100g/h (1.85 moles/h). Operating conditions were 120°C and 4.24 MPa. Liquid product was collected from the unit for 24 hours under steady state conditions.

The two-phase liquid product was allowed to stand at ambient temperature and the two layers were separated. A sample of the top layer (841g) was stripped to remove lights, solvent, etc. and the residual liquid (682g) had the analysis

| | |
|---|---|
| Number Average MW | 1083 |
| Weight Average MW | 1023 |
| Dispersity | 1.9 |
| Viscosity | 2076 cs/25°C |
| Hydroxyl Number | 2.6 meq KOH/g |

A sample of the heavier layer (6247g) was likewise stripped to remove lights, solvent, etc., and the residual liquid (351g) had the analysis

| | |
|---|---|
| Number Average MW | 228 |
| Weight Average MW | 572 |
| Dispersity | 2.5 |
| Viscosity | 1334 cs/25°C |
| Hydroxyl Number | 9.4 meq KOH/g |

EXAMPLE 3

The autoclave used in Example 1 was charged with ethylene glycol monomethyl ether acetate, and then heated to 120°C under a pressure of 4.24 MPa. A mixture of 50% hydrogen peroxide aqueous solution (2 parts) and ethylene glycol monomethyl ether acetate (3 parts) was then fed in continuously at a rate of 300g/h (1.76 moles $H_2O_2$/h), and when the system was lined out, a second feed of 1,3-butadiene was also introduced simultaneously at a rate of 100g/h (1.85 moles/h). After a few hours on stream, typical liquid product was collected under these steady state conditions for 30 hours.

The two-phase liquid product was allowed to stand and the two layers were separated. A sample (1462g) of the top layer was stripped to remove lights, solvent, etc., and the residue was a water-white liquid (1026g) having the analysis:

| | |
|---|---|
| Number Average mw | 1183 |
| Weight Average mw | 2154 |
| Dispersity | 1.8 |
| Viscosity | 2900 cs/25°C |
| Hydroxyl Number | 1.6 meq KOH/g |

$^{13}C$ NMR analysis of this product shows the total carbon association with the three major functionalities of this material, viz. olefin OH and aliphatic, to be about 47, 6.6 and 46%, respectively.

A sample (4505g) of the heavier layer was likewise stripped to remove lights, solvent, etc., and the residual liquid (633g) had the analysis:

| | |
|---|---|
| Number Average mw | 246 |
| Weight Average mw | 543 |
| Dispersity | 2.2 |
| Viscosity | 2998 cs/25°C |
| Hydroxyl Number | 8.7 meq KOH/g |

EXAMPLE 4

The autoclave reactor system of Example 1, was charged with a mixture of 50% hydrogen peroxide solution (2 parts) and 1,2-propylene glycol monomethyl ether acetate (3 parts) at a rate of 300g/h (1.76 moles $H_2O_2$/h, plus 1,3-butadiene at 100g/h (1.85 moles/h). Operating conditions were 120°C and 4.24 MPa. Liquid product was collected from the unit for 24 hours under steady state conditions.

The two-phase liquid product was allowed to stand at ambient temperature and the two layers were separated. A sample of the top layer (1043g) was stripped to remove lights, solvent, etc., and the residual liquid (637g) had the analysis:

| | |
|---|---|
| Number Average mw | 1534 |
| Weight Average mw | 2689 |
| Dispersity | 1.8 |
| Hydroxyl Number | 10.5 meq KOH/g |

A sample of the heavier layer (6865g) was likewise stripped to remove lights, solvent, etc., and the residual liquid (708g) had the analysis:

| | |
|---|---|
| Number Average mw | 251 |
| Weight Average mw | 643 |
| Dispersity | 2.6 |
| Viscosity | 10.5 cs.25°C |
| Hydroxyl Number | 8.7 meq KOH/g |

## Claims

1. A process for the preparation of hydroxyl-terminated diene oligomers by polymerizing a 1,3-diene in the presence of an aqueous solution of hydrogen peroxide, characterized in that a glycol monoalkyl ether or glycol monoalkyl ether carboxylate is employed as solvent.

2. A process according to claim 1 characterized in that the diene is 1,3-butadiene.

3. A process according to Claim 1 or 2 characterised in that the aqueous hydrogen peroxide solution has a concentration of 10 to 80%.

4. A process according to any one of claims 1 to 3 characterized in that the molar ratio of hydrogen peroxide feed to 1,3-diene is from 100:1 to 1:100.

5. A process according to any one of claims 1 to 4 characterized in that polymerization is conducted at a temperature from 50 to 200 ° C, and a pressure of 0.1 to 35 MPa.

6. A process according to any one of claims 1 to 5 characterized in that the solvent is a glycol ether of the formula

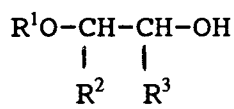

wherein $R^1$ is $C_1$-$C_{10}$ alkyl, and $R_2$ and $R^3$ are each hydrogen or methyl.

7. A process according to any one of claims 1 to 6 characterized in that the glycol ether is propylene glycol t-butyl ether, 2-methoxyethanol, 2-ethoxyethanol, 2-isopropoxyethanol, 2-t-butoxyethanol or diethylene glycol monobutyl ether.

8. A process according to any one of claims 1 to 5 characterized in that the solvent is a glycol monoalkyl ether carboxylate having the formula

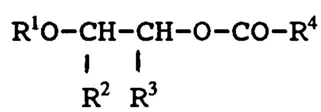

wherein $R^1$, $R^2$ and $R^3$ have the meanings given in claim 6, and $R^4$ is $C_1$-$C_{10}$ alkyl.

9. A process according to claim 8 characterized in that the solvent is an alkylene glycol monoalkyl ether acetate.

10. A process according to claim 9 characterized in that the solvent is ethylene glycol monomethyl ether acetate or propylene glycol monomethyl ether acetate.

European Patent Office

# EUROPEAN SEARCH REPORT

Application Number

EP 91 30 5793

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | US-A-3 673 168 (O.W.BURKE ET AL.)<br>* claim 12 *<br>* column 11, line 49 - column 11, line 50 *<br>* examples 27-28 *<br>--- | 1-8 | C08F36/04<br>C08F4/30<br>C08F2/06 |
| X | CHEMICAL ABSTRACTS, vol. 100, no. 20, 1983, Columbus, Ohio, US;<br>abstract no. 157969D,<br>B.VERUOVIC: 'PREPARATION OF LIQUID POLYBUTADIENE WITH TERMINAL HYDROXYL GROUPS'<br>& SB.VYS.SK.CHEM-TECHNOL.PRAZE,[ODDIL] S, S 9, 217-36<br>* abstract, line 3 *<br>--- | 1-8 | |
| A | GB-A-957 788 (TEXAS BUTADIENE AND CHEMICAL INTERNATIONAL LIMITED)<br>* claims 1,7 *<br>* page 2, line 112 *<br>----- | 1 | |
| | | | TECHNICAL FIELDS SEARCHED (Int. Cl.5 ) |
| | | | C08F |

The present search report has been drawn up for all claims

| Place of search | Date of completion of the search | Examiner |
|---|---|---|
| THE HAGUE | 31 JANUARY 1992 | VAN HUMBEECK F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)